# FASCICULE DE BREVET EUROPEEN

(11) **EP 1 567 097 B1**
(45) Date de publication et mention de la délivrance du brevet: **20.02.2008**
(21) Numéro de dépôt: 03778449.3
(22) Date de dépôt: 24.10.2003
(51) Int. Cl.: A61F 2/44

(54) **CAGE INTERVERTEBRALE A LAME D'ANCRAGE MEDIANE**
ZWISCHENWIRBELKÄFIG MIT MITTIGEM VERANKERUNGSBLATT
INTERVERTEBRAL CAGE WITH MEDIAL FIXING PLATE

(30) Priorité: 12.11.2002 FR 0214080
(43) Date de publication de la demande: 31.08.2005
(73) Titulaire: Razian, Hassan, 94240 l'Hay les Roses (FR)
(72) Inventeur: Razian, Hassan, 94240 l'Hay les Roses (FR)
(74) Mandataire: Flavenot, Bernard
(86) Numéro de dépôt international: PCT/FR2003/003149
(87) Numéro de publication internationale: WO 2004/043306

(56) Documents cités:
- DE-C- 19 816 832
- US-A- 5 683 394
- US-A- 6 102 949
- US-B1- 6 371 987

## Description

La présente invention concerne les cages intervertébrales qui trouvent une application particulièrement avantageuse, mais non exclusivement, pour le traitement du rachis dégénératif, et plus particulièrement un perfectionnement à des cages intervertébrales déjà connues.

En effet, des cages intervertébrales, notamment pour le traitement du rachis dégénératif, sont déjà connues. Une telle cage est par exemple décrite dans le document EP-A-1 104 665. Elle comporte essentiellement une entretoise en forme de disque comprenant deux faces de base opposées sensiblement planes et parallèles et une paroi latérale reliant les deux faces de base. Cette entretoise est apte à être disposée entre les faces en regard des deux corps vertébraux respectivement de deux vertèbres consécutives, en remplacement du disque endommagé situé entre ces deux vertèbres, les deux faces de base de l'entretoise étant placées au contact des corps vertébraux. L'entretoise peut en outre comporter une cavité ouverte dans laquelle il est possible de placer un greffon osseux ou analogue dans le but de souder entre eux les deux corps vertébraux par ostéosynthèse. La cage comprend aussi au moins une lame, comportant deux extrémités opposées conformées en biseau, et des moyens pour déplacer cette lame par rapport à une première des deux parties de paroi latérale de façon que la lame soit apte à prendre deux positions, une première position dans laquelle la lame est entièrement située dans l'espace compris entre les deux premier et second plans contenant les deux faces de base de l'entretoise, et une seconde position dans laquelle les deux extrémités opposées de la lame émergent de part et d'autre de cet espace.

Dans les cages décrites ci-dessus, la lame d'ancrage est associée à une entretoise sur une face de cette dernière. Une telle réalisation nécessite un certain nombre d'éléments pour pouvoir obtenir de façon relativement aisée la rotation de la lame par rapport à l'entretoise. Pour tenter de minimiser le nombre de ces éléments, une solution a été tentée, celle qui consiste à positionner la lame d'ancrage sensiblement dans la partie médiane de l'entretoise, comme par exemple dans le mode de réalisation qui est décrit dans le WO 01/01894. La solution adoptée pour la réalisation de cette cage n'a cependant pas donné entière satisfaction, essentiellement par le fait que l'intégration de la lame dans la partie médiane de l'entretoise était encore trop complexe et rendait relativement compliquée l'implantation de la cage entre deux vertèbres.

Pour tenter de pallier les inconvénients mentionnés ci-dessus, il a aussi été réalisé une cage intervertébrale comme celle décrite par exemple dans le US-B1-6 371 987. Cette cage répond de façon générale aux exigences des praticiens mais présente encore des inconvénients notamment pour sa réalisation.

Les préambules des revendications 1 et 8 sont basés sur cette cage.

La présente invention a donc pour but de réaliser un perfectionnement aux cages intervertébrales du type de celles définies ci-dessus, qui présente une structure plus facile à réaliser et à assembler, et qui permet une implantation beaucoup plus aisée de la cage entre deux vertèbres.

Plus précisément, la présente invention a pour objet une cage intervertébrale pour le traitement du rachis dégénératif apte à être interposée entre deux vertèbres consécutives, comprenant :
- un bloc,
- au moins une lame d'ancrage comportant un pivot définissant un premier axe de rotation, et au moins une ailette montée solidaire dudit pivot suivant sensiblement un premier plan faisant avec ledit premier axe un angle α non nul, et
- des moyens pour monter le pivot en rotation par rapport au dit bloc, comportant une percée réalisée dans le bloc suivant un second axe, une saignée réalisée dans le bloc suivant sensiblement un second plan faisant avec le second axe un angle γ sensiblement égal à l'angle α, la saignée étant en outre réalisée de façon qu'elle ait, avec la percée, une partie commune apte à contenir le pivot, et des moyens pour associer le pivot en rotation avec le bloc quand le pivot est positionné dans ladite partie commune et de façon que lorsque, dans cette position, le pivot subit une rotation d'une amplitude donnée par rapport au bloc, la lame d'ancrage soit apte à prendre au moins deux première et seconde positions, la première position étant celle dans laquelle l'ailette est totalement contenue dans la saignée, et la seconde position étant celle dans laquelle une portion de l'extrémité de l'ailette émerge de ladite saignée,
caractérisée par le fait que, le pivot étant constitué par un second arbre de rotation ayant pour axe le premier axe, les moyens pour associer le pivot en rotation avec le bloc comportent un second palier ouvert en direction de l'ouverture de ladite saignée située en surface du bloc, ledit second palier ouvert étant réalisé en bordure de ladite partie commune de façon qu'il soit centré sur ledit second axe, les diamètres du second palier ouvert et du second arbre de rotation étant sensiblement égaux, le diamètre du second arbre de rotation et du second palier ouvert étant supérieur à la dimension diamétrale minimale de la section transversale de la percée.

Selon une variante, la cage intervertébrale est caractérisée en ce que
le pivot est constitué par un premier palier en creux, et les moyens pour associer le pivot en rotation avec le bloc comportent un premier arbre de rotation monté avec des moyens d'indexation en rotation dans ledit premier palier en creux, et des moyens pour monter ledit premier arbre de rotation en coopération avec ledit bloc.

D'autres caractéristiques et avantages de l'invention apparaîtront au cours de la description suivante donnée en regard des dessins annexés à titre illustratif mais nullement limitatif, dans lesquels :
La figure 1 représente une vue en perspective cavalière, en demi-coupe et en éclaté, d'un premier mode de réalisation schématique de la cage intervertébrale selon l'invention,
La figure 2 représente une vue de dessus d'un deuxième mode de réalisation préféré de la cage selon l'invention, et
La figure 3 représente une vue partielle en coupe d'un autre troisième mode de réalisation possible de la cage selon l'invention.

Il est bien précisé que, sur les figures, les mêmes références désignent les mêmes éléments, quelle que soit la figure sur laquelle elles apparaissent et quelle que soit la forme de représentation de ces éléments.

De même, si des éléments ne sont pas spécifiquement référencés sur l'une des figures, leurs références peuvent être aisément retrouvées en se reportant à une autre figure.

Le Demandeur tient aussi à préciser que les figures représentent trois modes de réalisation de l'objet selon l'invention, mais qu'il peut exister d'autres modes de réalisation qui répondent à la définition de cette invention.

Il précise en outre que, lorsque, selon la définition de l'invention, l'objet de l'invention comporte "au moins un" élément ayant une fonction donnée, le mode de réalisation décrit peut comporter plusieurs de ces éléments.

Il précise aussi que, si les modes de réalisation de l'objet selon l'invention tel qu'illustrés comportent plusieurs éléments de fonction identique et que si, dans la description, il n'est pas spécifié que l'objet selon cette invention doit obligatoirement comporter un nombre particulier de ces éléments, l'objet de l'invention pourra être défini comme comportant "au moins un" de ces éléments.

La présente invention concerne une cage intervertébrale perfectionnée pour, notamment, le traitement du rachis dégénératif.

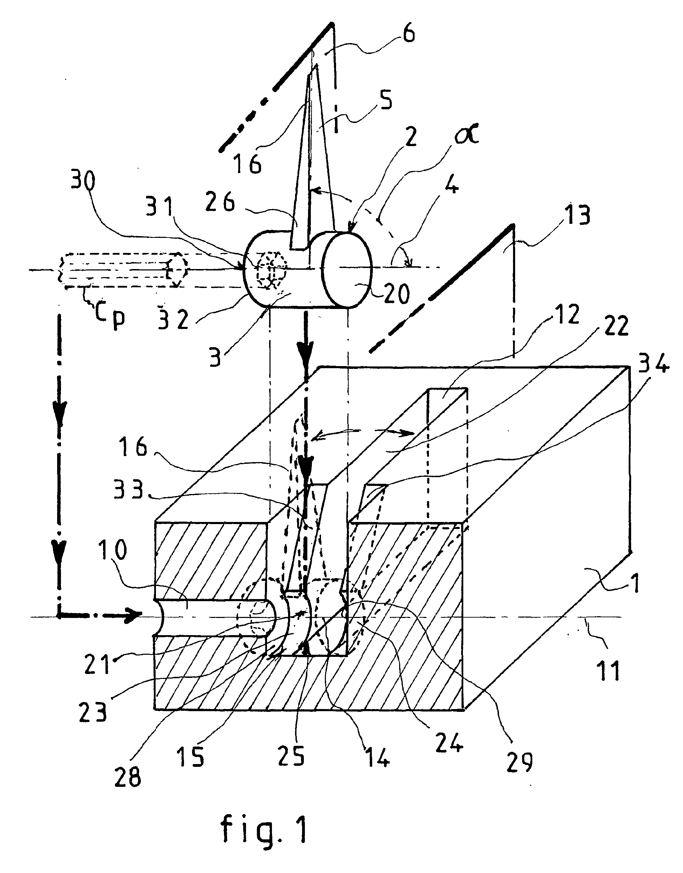

La figure 1, sous forme schématique fonctionnelle, un mode de réalisation d'une cage intervertébrale selon l'invention.

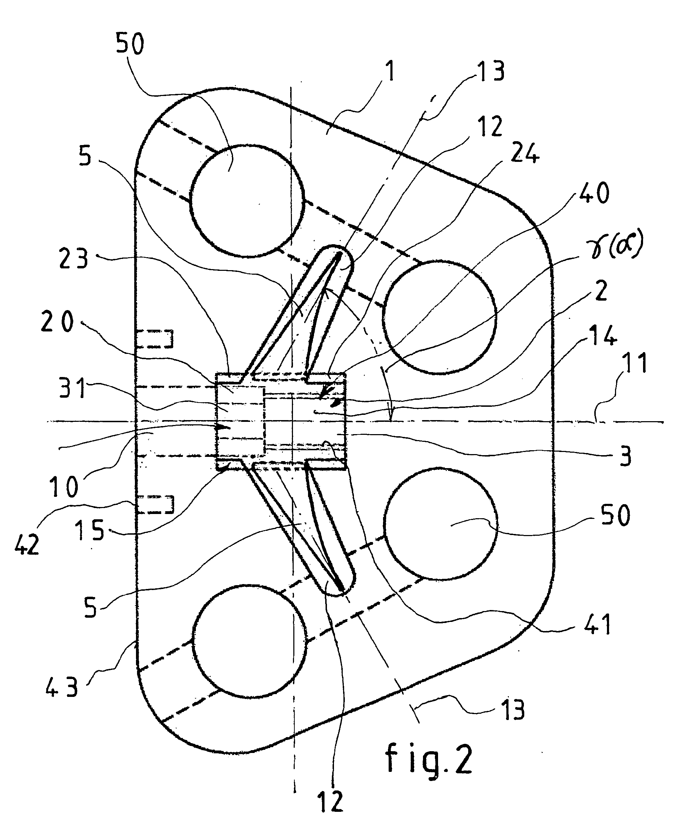

Cette cage comprend un bloc 1 d'une forme assimilable à celle d'un parallélépipède rectangle ou, plus généralement, qui peut être inscrite dans un parallélépipède rectangle. Elle peut notamment être comme celle illustrée sur la figure 2.

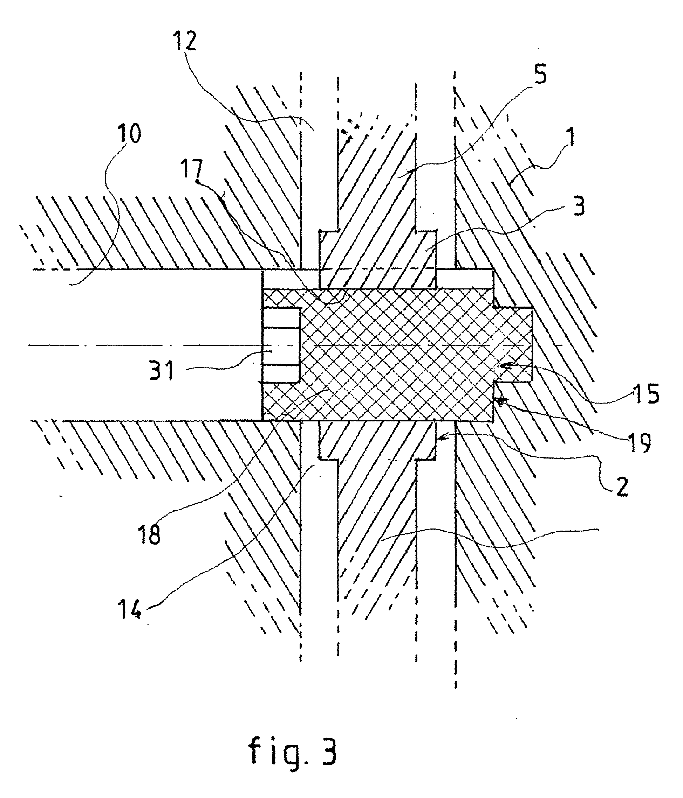

La cage comporte aussi au moins une lame d'ancrage 2 comportant un pivot 3 définissant un premier axe de rotation 4, et au moins une ailette 5 montée solidaire du pivot 3 suivant sensiblement un premier plan 6 faisant avec le premier axe 4 un angle α non nul, et avantageusement deux ailettes comme dans les réalisations selon les figures 2 et 3 pour s'ancrer dans les deux vertèbres consécutives entre lesquelles la cage est destinée à être implantée.

Elle comporte des moyens pour monter le pivot 3 en rotation par rapport au bloc 1, qui comprennent une percée 10 réalisée dans le bloc suivant un second axe 11, une saignée 12 réalisée dans le bloc suivant sensiblement un second plan 13 faisant avec le second axe 11 un angle γ sensiblement égal à l'angle α, la saignée 12 étant en outre réalisée de façon qu'elle ait, avec la percée 10, une partie commune 14 apte à contenir le pivot 3, et des moyens 15 pour associer le pivot 3 en rotation avec le bloc 1 quand le pivot est positionné dans la partie commune 14 et de façon que lorsque, dans cette position, le pivot subit une rotation d'une amplitude donnée par rapport au bloc, la lame d'ancrage 2 soit apte à prendre au moins deux première et seconde positions, la première position étant celle dans laquelle l'ailette 5 est totalement contenue dans la saignée 12, et la seconde position étant celle dans laquelle une portion 16 de l'extrémité de l'ailette émerge de la saignée.

Il est précisé que, dans le mode de réalisation selon la figure 1, la lame d'ancrage 2 ne comporte qu'une ailette 5. Dans ce cas, la saignée 12 peut avoir la forme telle qu'illustrée sur cette figure 1. Dans le cas où la lame d'ancrage comporte deux ailettes sensiblement symétriques, comme dans le mode de réalisation selon la figure 2, à chaque ailette correspond au moins une saignée 12 comme celle illustrée sur la figure 1. Cependant, dans un mode de réalisation avantageux notamment sur le plan de l'usinage, les deux saignées correspondant aux deux ailettes seront réalisées suivant une seule saignée traversant de part en part le bloc 1 comme illustré sur la figure 2, cette réalisation permettant en outre de faire pivoter la lame d'ancrage dans un sens ou dans l'autre pour obtenir les résultats définis ci-après.

Dans le mode de réalisation illustré sur la figure 1, les angles α et γ sont sensiblement égaux à 90 degrés mais ils seront avantageusement choisis à une valeur inférieure, par exemple 70 degrés pour permettre un accrochage rétentif de la cage avec les deux vertèbres.

Dans une réalisation possible comme celle illustrée sur la figure 3, le pivot 3 est constitué par un premier palier en creux 17, avantageusement en forme de manchon dont la paroi intérieure est cylindrique de révolution. Dans ce cas, les moyens 15 pour associer le pivot 3 en rotation avec le bloc 1 comportent un premier arbre de rotation 18 monté avec des moyens d'indexation en rotation dans le premier palier en creux 17, et des moyens 19 pour monter le premier arbre de rotation 18 en coopération avec le bloc 1. Comme illustré sur la figure 3, ces moyens 19 peuvent être constitués par un assemblage du type à clavette par exemple solidaire du pivot 3 coopérant avec une rainure correspondante réalisée dans le premier arbre de rotation 18. De plus, ce premier arbre de rotation 18 a une section transversale sensiblement complémentaire de celle de la percée 10 pour qu'il puisse y être introduit à friction relativement dure.

Ce mode de réalisation est relativement intéressant sur le plan de sa structure et pour l'implantation de la cage.

Cependant, à ce mode de réalisation selon la figure 3, est préféré un mode de réalisation comme celui illustré sur les figures 1 et 2, qui présente deux avantages essentiels par rapport au mode de réalisation selon la figure 3, à savoir qu'il nécessite moins d'éléments constitutifs, deux au lieu de trois, que l'assemblage de ces éléments se fait très facilement, automatiquement.

Dans les modes de réalisation selon les figures 1 et 2, le pivot 3 est constitué par un second arbre de rotation 20 ayant pour axe le premier axe 4, et les moyens 15 pour associer le pivot en rotation avec le bloc comportent un second palier 21 ouvert en direction de l'ouverture 22 de la saignée 12 située en surface du bloc 1, le second palier ouvert étant réalisé en bordure de la partie commune 14 de façon qu'il soit centré sur le second axe 11, les diamètres du second palier ouvert 21 et du second arbre de rotation 20 étant sensiblement égaux.

Avantageusement, le diamètre du second arbre de rotation 20 et du second palier ouvert 21 est supérieur à la dimension diamétrale minimale de la section transversale de la percée 10. De cette façon, quand le pivot est disposé dans le second palier ouvert 21, il est maintenu entre les deux épaulements formés par le bord 28 de l'extrémité de la percée 10 qui débouche dans la partie commune 14 et par la paroi 29 de la saignée 12.

De façon préférentielle, le second palier ouvert 21 est constitué de deux surfaces cylindriques ouvertes 23, 24 séparées par un espace libre 25 d'une largeur au moins égale à l'épaisseur maximale de l'ailette 5 prise au niveau de sa partie 26 qui est solidaire du pivot 3.

Dans un mode de réalisation avantageux, ce second palier ouvert 21 est un palier de rétention. Dans ce cas, au moins l'une des deux surfaces cylindriques ouvertes 23, 24, et avantageusement les deux, est définie sur un angle supérieur à 180 degrés, mais cependant très peu supérieur à 180 degrés.

De plus, selon le mode de réalisation décrit ci-dessus, les deux surfaces cylindriques ouvertes 23, 24 sont reliées à la surface du bloc 1 sur laquelle débouche la saignée 12, respectivement par deux rampes 33, 34 qui forment deux rampes de guidage pour l'introduction du second arbre de rotation 20 dans le second palier en creux 21. Sur la figure 1, ces deux rampes 33, 34, que l'on retrouve bien entendu de façon symétrique dans l'autre moitié du bloc 1 non représentée, forment un entonnoir dont l'angle au sommet est relativement grand afin de faire ressortir la fonction de guidage de ces deux rampes. Cependant, dans la pratique, comme illustré sur la figure 1, cet angle au sommet a une valeur très faible.

La cage intervertébrale comporte en outre des moyens 30 pour entraîner en rotation le pivot 3 autour du second axe 11 de façon que la lame d'ancrage 2 soit apte à prendre ses deux première et seconde positions définies ci-avant.

Ces moyens 30 sont par exemple constitués d'un logement en creux 31 à section transversale polygonale réalisé dans la face 32 du second arbre de rotation 20 qui est en regard de la percée 10 quand le second arbre de rotation 20 est monté en rotation dans le second palier ouvert 21, ce logement en creux 31 étant sensiblement centré sur le premier axe 4 et ayant une section transversale inférieure à celle de la percée 10.

Dans une réalisation avantageuse, la cage intervertébrale comporte un orifice 40 dans la paroi duquel est réalisé un taraudage 41, qui est réalisé dans le second arbre de rotation 20 en étant centré sur le premier axe 4 et en débouchant dans le fond du logement en creux 31, le diamètre de l'orifice taraudé 40 étant inférieur à la section transversale de ce logement en creux, et des moyens 42 pour indexer la position d'un ancillaire par rapport au bloc 1 réalisés sur la face 43 du bloc sur laquelle débouche la percée 10. Ces moyens 42 sont par exemple constitués de deux encoches, ou plus, en sachant que l'ancillaire doit comporter des ergots aptes à être enfichés dans les encoches 42 et une tige filetée apte à se visser dans l'orifice taraudé 40 lorsque les ergots sont enfichés dans les encoches.

Les éléments de la cage intervertébrale telle que décrite ci-dessus en regard plus particulièrement des figures 1 et 2 s'assemblent comme suit et la cage s'utilise de la façon suivante :

Tout d'abord, il est précisé que le bloc 1 et la lame d'ancrage 2 sont usinés de façon classique pour avoir les caractéristiques structurelles décrites ci-avant. Ces deux éléments étant réalisés, la lame d'ancrage 2 est présentée, par le pivot 20, dans l'entonnoir formé par les rampes 33, 34. Elle est poussée en force jusqu'à ce que le pivot vienne s'encliqueter dans le second palier ouvert 21. Dès que le pivot est positionné dans ce second palier ouvert, il y est parfaitement maintenu par les deux épaulements 28, 29 et par les deux surfaces cylindriques ouvertes 23, 24 définies sur un angle supérieur à 180 degrés.

Lorsque la lame d'ancrage 2 a pris une position comme celle illustrée en traits interrompus sur la figure 1, qui correspond à sa seconde position définie auparavant, au moyen par exemple d'une clé à section polygonale Cp, représentée en traits interrompus sur la figure 1, complémentaire du logement 31 introduite dans ce logement via la percée 10, la lame d'ancrage 2 est amenée dans sa première position comme représentée sur la figure 2. Dans cette position, les deux ailettes 5 de la lame sont complètement escamotées, entièrement contenues dans les saignées 12.

De façon connue, le praticien introduit alors la cage selon l'invention entre deux vertèbres consécutives en remplacement du disque intervertébral, la lame d'ancrage étant dans sa première position. Pour ce faire, le praticien utilise l'ancillaire décrit ci-avant.

Au moyen de la clé à section polygonale Cp introduite dans le logement 31 via la percée 10, le praticien fait alors passer la lame d'ancrage de sa première position à sa seconde position, les portions d'extrémité 16 des ailettes 5 s'implantant dans les parties osseuses des deux vertèbres, de la même façon que dans le cas des cages intervertébrales du même type selon l'art antérieur.

Pour déterminer la seconde position de la lame d'ancrage et favoriser la pose de la cage par le praticien, la cage peut comporter en outre, par exemple un premier cliquet constitué de façon connue en elle-même d'un ergot réalisé sur le pivot et d'une rainure réalisée dans le bloc 1 qui coopèrent l'un dans l'autre, par exemple par déformation, quand la lame d'ancrage arrive dans sa seconde position. La cage peut aussi comporter un second cliquet pour définir la première position de la lame d'ancrage, le même ergot pouvant d'ailleurs être commun aux deux cliquets.

Il est précisé que la cage selon l'invention peut comporter en outre d'autres caractéristiques que celles définies ci-dessus, par exemple des trous 50 de réception de greffons osseux, comme représenté sur la figure 2. Ces autres caractéristiques n'ont pas été décrites ici car elles n'entrent pas dans le champ de la présente invention.

## Revendications

1. Cage intervertébrale pour le traitement du rachis dégénératif apte à être interposée entre deux vertèbres consécutives, comprenant :
• un bloc (1),
• au moins une lame d'ancrage (2) comportant un pivot (3) définissant un premier axe de rotation (4), et au moins une ailette (5) montée solidaire dudit pivot (3) suivant sensiblement un premier plan (6) faisant avec ledit premier axe (4) un angle α non nul, et
• des moyens pour monter le pivot (3) en rotation par rapport au dit bloc (1), comportant une percée (10) réalisée dans le bloc (1) suivant un second axe (11), une saignée (12) réalisée dans le bloc suivant sensiblement un second plan (13) faisant avec le second axe (11) un angle γ sensiblement égal à l'angle α, la saignée (12) étant en outre réalisée de façon qu'elle ait, avec la percée (10), une partie commune (14) apte à contenir le pivot (3), et des moyens (15) pour associer le pivot (3) en rotation avec le bloc (1) quand le pivot est positionné dans ladite partie commune (14) et de façon que lorsque, dans cette position, le pivot subit une rotation d'une amplitude donnée par rapport au bloc, la lame d'ancrage (2) soit apte à prendre au moins deux première et seconde positions, la première position étant celle dans laquelle l'ailette (5) est totalement contenue dans la saignée (12), et la seconde position étant celle dans laquelle une portion (16) de l'extrémité de l'ailette émerge de ladite saignée,
**caractérisée par le fait que**, le pivot (3) étant constitué par un second arbre de rotation (20) ayant pour axe le premier axe (4), les moyens (15) pour associer le pivot en rotation avec le bloc comportent un second palier (21) ouvert en direction de l'ouverture (22) de ladite saignée (12) située en surface du bloc (1), ledit second palier ouvert étant réalisé en bordure de ladite partie commune (14) de façon qu'il soit centré sur ledit second axe (11), les diamètres du second palier ouvert (21) et du second arbre de rotation (20) étant sensiblement égaux, le diamètre du second arbre de rotation (20) et du second palier ouvert (21) étant supérieur à la dimension diamétrale minimale de la section transversale de la percée (10).

2. Cage intervertébrale selon la revendication 1, **caractérisée par le fait que** le second palier ouvert (21) est constitué de deux surfaces cylindriques ouvertes (23, 24) séparées par un espace libre (25) d'une largeur au moins égale à l'épaisseur maximale de l'ailette (5) prise au niveau de sa partie (26) qui est solidaire du pivot (3).

3. Cage intervertébrale selon la revendication 2, **caractérisée par le fait que** le second palier ouvert (21) est un palier de rétention.

4. Cage intervertébrale selon la revendication 3, **caractérisée par le fait qu'**au moins l'une des deux surfaces cylindriques ouvertes (23, 24) est définie sur un angle supérieur à 180 degrés.

5. Cage intervertébrale selon l'une des revendications 1 à 4, **caractérisée par le fait qu'**elle comporte des moyens (30) pour entraîner en rotation ledit pivot (3) autour dudit second axe (11) de façon que ladite lame d'ancrage (2) soit apte à prendre ses deux dites première et seconde positions.

6. Cage intervertébrale selon la revendication 5, **caractérisée par le fait que** les moyens (30) pour entraîner en rotation ledit pivot (3) autour dudit second axe (11) comportent un logement en creux (31) à section transversale polygonale réalisé dans la face (32) du second arbre de rotation (20) qui est en regard de la percée (10) quand ledit second arbre de rotation (20) est monté en rotation dans le second palier ouvert (21), ledit logement en creux (31) étant sensiblement centré sur ledit premier axe (4) et ayant une section transversale inférieure à celle de ladite percée (10).

7. Cage intervertébrale selon la revendication 6, **caractérisée par le fait qu'**elle comporte un orifice (40) comportant un taraudage (41), ledit orifice étant réalisé dans le second arbre de rotation (20) en étant centré sur le premier axe (4) et en débouchant dans le fond dudit logement en creux (31), le diamètre dudit orifice taraudé (40) étant inférieur à la section transversale dudit logement en creux (31), et des moyens (42) pour indexer la position d'un ancillaire par rapport au bloc (1) réalisés sur la face (43) du bloc sur laquelle débouche ladite percée (10).

8. Cage intervertébrale pour le traitement du rachis dégénératif apte à être interposée entre deux vertèbres consécutives, comprenant :
• un bloc (1),
• au moins une lame d'ancrage (2) comportant un pivot (3) définissant un premier axe de rotation (4), et au moins une ailette (5) montée solidaire dudit pivot (3) suivant sensiblement un premier plan (6) faisant avec ledit premier axe (4) un angle α non nul, et
• des moyens pour monter le pivot (3) en rotation par rapport au dit bloc (1), comportant une percée (10) réalisée dans le bloc (1) suivant un second axe (11), une saignée (12) réalisée dans le bloc suivant sensiblement un second plan (13) faisant avec le second axe (11) un angle γ sensiblement égal à l'angle α, la saignée (12) étant en outre réalisée de façon qu'elle ait, avec la percée (10), une partie commune (14) apte à contenir le pivot (3), et des moyens (15) pour associer le pivot (3) en rotation avec le bloc (1) quand le pivot est positionné dans ladite partie commune (14) et de façon que lorsque, dans cette position, le pivot subit une rotation d'une amplitude donnée par rapport au bloc, la lame d'ancrage (2) soit apte à prendre au moins deux première et seconde positions, la première position étant celle dans laquelle l'ailette (5) est totalement contenue dans la saignée (12), et la seconde position étant celle dans laquelle une portion (16) de l'extrémité de l'ailette émerge de ladite saignée,
**caractérisée par le fait que**, le pivot (3) étant constitué par un premier palier en creux (17), les moyens (15) pour associer le pivot (3) en rotation avec le bloc (1) comportent un premier arbre de rotation (18) monté avec des moyens d'indexation en rotation dans ledit premier palier en creux (17), et des moyens (19) pour monter ledit premier arbre de rotation (18) en coopération avec ledit bloc (1).

## Claims

1. An intervertebral cage for treating degeneration of the spine and suitable for being interposed between two consecutive vertebrae, the cage comprising:
· a block (1);
· at least one anchor member (2) comprising a pivot (3) defining a first axis of rotation (4), and at least one blade (5) secured to said pivot (3) and occupying substantially a first plane (6) making a non-zero angle α relative to said first axis (4); and
· mean for mounting the pivot (3) to turn relative to said block (1), said means comprising a hole (10) made in the block (1) along a second axis (11), a slot (12) made in the block substantially in a second plane (13) making an angle γ substantially equal to the angle α relative to the second axis (11), the slot (12) also being made in such a manner that, together with the hole (10), it has a common portion (14) suitable for containing the pivot (3), and means (15) for associating the pivot (3) to turn relative to the block (1) when the pivot is in position in said common portion (14) and in such a manner that when, in said position, the pivot is turned through a given amplitude relative to the block, the anchor member (2) is suitable for taking up at least a first position and a second position, the first position being that in which the blade (5) is fully contained within the slot (12), and the second position being that in which a portion (16) of the end of the blade emerges from said slot;
the cage being **characterized by** the fact that for the pivot (3) being constituted by a second rotary shaft (20) having the first axis (4) as its axis, the means (15) for associating the pivot in rotation with the block comprises a second bearing (21) that is open towards the opening (22) of said slot (12) situated in the surface of the block (1), said open second bearing being made in the margin of said common portion (14) in such a manner as to be centered on said second axis (11), the diameters of the open second bearing (21) and of the second rotary shaft (20) being substantially equal, the diameter of the second rotary shaft (20) and of the open second bearing (21) being greater than the minimum diametral dimension of the cross-section of the hole (10).

2. An intervertebral cage according to claim 1, **characterized by** the fact that the open second bearing (21) is constituted by two open cylindrical surfaces (23, 24) separated by an empty space (25) of width that is not less than the maximum thickness of the blade (5) in its portion (26) that is secured to the pivot (3).

3. An intervertebral cage according to claim 2, **characterized by** the fact that the open second bearing (21) is a retention bearing.

4. An intervertebral cage according to claim 3, **characterized by** the fact that at least one of the two open cylindrical surfaces (23, 24) is defined over an angle greater than 180°.

5. An intervertebral cage according to any one of claims 1 to 4, **characterized by** the fact that it includes means (30) for turning said pivot (3) about said second axis (11) in such a manner that said anchor member (2) is suitable for taking up said first position and said second position.

6. An intervertebral cage according to claim 5, **characterized by** the fact that the means (30) for turning said pivot (3) about said second axis (11) comprise a socket (31) of polygonal cross-section made in the face (32) of the second rotary shaft (20) that faces the hole (10) when said second rotary shaft (20) is mounted to rotate in the open second bearing (21), said socket (31) being centered substantially on said first axis (4) and being of cross-section smaller than that of said hole (10).

7. An intervertebral cage according to claim 6, **characterized by** the fact that it includes an orifice (40) having tapping (41), said orifice being formed in the second rotary shaft (20) being centered on the first axis (4) and opening out into the end of said hollow recess (31), the diameter of said tapped orifice (40) being less than the cross-section of said socket (31), and means (42) for indexing the position of an ancillary relative to the block (1) formed in the face (43) of the block into which said hole (10) opens out.

8. An intervertebral cage for treating degeneration of the spine and suitable for being interposed between two consecutive vertebrae, the cage comprising:
· a block (1);
· at least one anchor member (2) comprising a pivot (3) defining a first axis of rotation (4), and at least one blade (5) secured to said pivot (3) and occupying substantially a first plane (6) making a non-zero angle α relative to said first axis (4); and
· means for mounting the pivot (3) to turn relative to said block (1), said means comprising a hole (10) made in the block (1) along a second axis (11), a slot (12) made in the block substantially in a second plane (13) making an angle γ substantially equal to the angle α relative to the second axis (11), the slot (12) also being made in such a manner that, together with the hole (10), it has a common portion (14) suitable for containing the pivot (3), and means (15) for associating the pivot (3) to turn relative to the block (1) when the pivot is in position in said common portion (14) and in such a manner that when, in said position, the pivot is turned through a given amplitude relative to the block, the anchor member (2) is suitable for taking up at least a first position and a second position, the first position being that in which the blade (5) is fully contained within the slot (12), and the second position being that in which a portion (16) of the end of the blade emerges from said slot;
**characterized by** the fact that, for the pivot (3) being constituted by a hollow first bearing (17), the means (15) for associating the pivot (3) in rotation with the block (1) comprise a first rotary shaft (18) mounted with rotary indexing means in said hollow first bearing (17), and means (19) for mounting said first rotary shaft (18) to co-operate with said block (1).

## Patentansprüche

1. Intervertebralkäfig für die Behandlung der Wirbelsäulendegeneration, der zwischen zwei aufeinanderfolgende Wirbel eingesetzt werden kann und umfasst:
- einen Block (1),
- wenigstens eine Verankerungsklinge (2) mit einem Drehzapfen (3), der eine erste Drehachse (4) definiert, und wenigstens einem Flügel (5), der an dem Drehzapfen (3) im Wesentlichen in einer ersten Ebene (6), die mit der ersten Achse (4) einen von Null verschiedenen Winkel α bildet, fest angebracht ist, und
- Mittel, um den Drehzapfen (3) drehbar in Bezug auf den Block (1) anzubringen, mit einem in dem Block (1) längs einer zweiten Achse (11) ausgebildeten Einschnitt (10), einer Furche (12), die in dem Block im Wesentlichen in einer zweiten Ebene (13) verwirklicht ist, die mit der zweiten Achse (11) einen Winkel γ bildet, der im Wesentlichen gleich dem Winkel α ist, wobei die Furche (12) außerdem in der Weise verwirklicht ist, dass sie mit dem Einschnitt (10) einen gemeinsamen Abschnitt (14) hat, der den Drehzapfen (3) enthalten kann, und Mitteln (15), die den Drehzapfen (3) mit dem Block (1) drehbar verbinden, wenn der Drehzapfen in dem gemeinsamen Abschnitt (14) positioniert ist, derart, dass der Drehzapfen in dieser Position eine Drehung mit einer gegebenen Amplitude in Bezug auf den Block ausführt, wobei die Verankerungsklinge (2) wenigstens eine erste und eine zweite Position einnehmen kann, wobei die erste Position jene ist, in der der Flügel (5) vollständig in der Furche (12) enthalten ist, und die zweite Position jene ist, in der ein Teil (16) des Endes des Flügels von der Furche vorsteht,
**dadurch gekennzeichnet, dass** dann, wenn der Drehzapfen (3) durch eine zweite Drehwelle (20) gebildet ist, die als Achse die erste Achse (4) besitzt, die Mittel (15) zum drehbaren Verbinden des Drehzapfens mit dem Block ein zweites Lager (21) aufweisen, das zu der Öffnung (22) der in der Oberfläche des Blocks (1) befindlichen Furche (12) offen ist, wobei das zweite offene Lager am Rand des gemeinsamen Abschnitts (14) verwirklicht ist, derart, dass es auf die zweite Achse (11) zentriert ist, wobei die Durchmesser des zweiten offenen Lagers (21) bzw. der zweiten Drehwelle (20) im Wesentlichen gleich sind und der Durchmesser der zweiten Drehwelle (20) bzw. des zweiten offenen Lagers (21) größer als die minimale diametrale Abmessung des transversalen Querschnitts des Einschnitts (10) ist.

2. Intervertebralkäfig nach Anspruch 1, **dadurch gekennzeichnet, dass** das zweite offene Lager (21) aus zwei offenen zylindrischen Oberflächen (23, 24) gebildet ist, die durch einen freien Zwischenraum (25) mit einer Breite, die wenigstens gleich der maximalen Dicke des Flügels (5) auf Höhe seines mit dem Drehzapfen (3) fest verbundenen Teils (26) ist, getrennt sind.

3. Intervertebralkäfig nach Anspruch 2, **dadurch gekennzeichnet, dass** das zweite offene Lager (21) ein Rückhaltelager ist.

4. Intervertebralkäfig nach Anspruch 3, **dadurch gekennzeichnet, dass** wenigstens eine der zwei offenen zylindrischen Oberflächen (23, 24) über einen Winkel von mehr als 180 Grad definiert ist.

5. Intervertebralkäfig nach einem der Ansprüche 1 bis 4, **dadurch gekennzeichnet, dass** er Mittel (30) zum rotatorischen Antreiben des Drehzapfens (3) um die zweite Achse (11) umfasst, derart, dass die Verankerungsklinge (2) ihre zwei Positionen, nämlich die so genannte erste und die so genannte zweite Position, einnehmen kann.

6. Intervertebralkäfig nach Anspruch 5, **dadurch gekennzeichnet, dass** die Mittel (30) zum rotatorischen Antreiben des Drehzapfens (3) um die zweite Achse (11) einen hohlen Aufnahmesitz (31) mit polygonförmigem Querschnitt umfassen, der in der Seite (32) der zweiten Drehwelle (20) verwirklicht ist, die sich gegenüber dem Einschnitt (10) befindet, wenn die zweite Drehwelle (20) in dem zweiten offenen Lager (21) drehbar angebracht ist, wobei der hohle Aufnahmesitz (31) auf die erste Achse (4) im Wesentlichen zentriert ist und einen Querschnitt besitzt, der kleiner als jener des Einschnitts (10) ist.

7. Intervertebralkäfig nach Anspruch 6, **dadurch gekennzeichnet, dass** er eine Öffnung (40) mit Gewinde (41) aufweist, wobei die Öffnung in der zweiten Drehwelle (20) verwirklicht ist, auf die erste Achse (4) zentriert ist und in den Boden des hohlen Aufnahmesitzes (31) mündet, wobei der Durchmesser der Gewindeöffnung (40) kleiner als der Querschnitt des hohlen Aufnahmesitzes (31) ist, und Mittel (42) umfasst, um die Position einer Hilfsvorrichtung in Bezug auf den Block (1) zu indexieren, die in der Seite (43) des Blocks verwirklicht sind, in die der Einschnitt (10) mündet.

8. Intervertebralkäfig für die Behandlung der Wirbelsäulendegeneration, der zwischen zwei aufeinanderfolgende Wirbel eingesetzt werden kann und umfasst:
- einen Block (1),
- wenigstens eine Verankerungsklinge (2) mit einem Drehzapfen (3), der eine erste Drehachse (4) definiert, und wenigstens einem Flügel (5), der an dem Drehzapfen (3) im Wesentlichen in einer ersten Ebene (6), die mit der ersten Achse (4) einen von Null verschiedenen Winkel α bildet, fest angebracht ist, und
- Mittel, um den Drehzapfen (3) drehbar in Bezug auf den Block (1) anzubringen, mit einem Einschnitt (10), der in dem Block (1) längs einer zweiten Achse (11) verwirklicht ist, einer Furche (12), die in dem Block im Wesentlichen in einer zweiten Ebene (13) verwirklicht ist, die mit der zweiten Achse (11) einen Winkel γ bildet, der im Wesentlichen gleich dem Winkel α ist, wobei die Furche (12) außerdem in der Weise verwirklicht ist, dass sie mit dem Einschnitt (10) einen gemeinsamen Abschnitt (14) besitzt, der den Drehzapfen (3) enthalten kann, und Mitteln (15), um den Drehzapfen (3) mit dem Block (1) drehbar zu verbinden, wenn der Drehzapfen in dem gemeinsamen Abschnitt (14) positioniert ist, und derart, dass der Drehzapfen in dieser Position eine Drehung mit einer gegebenen Amplitude in Bezug auf den Block ausführt, wobei die Verankerungsklinge (2) wenigstens zwei Positionen, nämlich eine erste Position und eine zweite Position, einnehmen kann, wobei die erste Position jene ist, in der der Flügel (5) vollständig in der Furche (12) enthalten ist, und die zweite Position jene ist, in der ein Teil (16) des Endes des Flügels von der Furche vorsteht,
**dadurch gekennzeichnet, dass** der Drehzapfen (3) durch ein erstes hohles Lager (17) gebildet ist, wobei die Mittel (15) zum drehbaren Verbinden des Drehzapfens (3) mit dem Block (1) eine erste Drehwelle (18), die zusammen mit Drehindexierungsmitteln in dem ersten hohlen Lager (17) angebracht ist, und Mittel (19) zum drehbaren Anbringen der ersten Welle (18) in Zusammenwirkung mit dem Block (1) umfassen.
